# EUROPEAN PATENT APPLICATION

(11) **EP 2 044 962 A1**
(43) Date of publication of application: **08.04.2009**
(21) Application number: 06812883.4
(22) Date of filing: 06.04.2006
(51) Int. Cl.: A61L 26/00, A61L 15/28, A61L 15/30

(54) **MONOLAYER WOUND COATING AND A METHOD FOR THE PRODUCTION THEREOF**

(71) Applicant: Gavrilyuk, Boris Karpovich, Moskovskaya obl. 142290 (RU); Gavrilyuk, Vadim Borisovich, Moscow 117513 (RU)
(72) Inventor: Gavrilyuk, Boris Karpovich, Moskovskaya obl. 142290 (RU); Gavrilyuk, Vadim Borisovich, Moscow 117513 (RU)
(74) Representative: Celestino, Marco
(86) International application number: PCT/RU2006/000166
(87) International publication number: WO 2007/114726

(57) **Abstract**

The invention can be used in medicine, in particular for treating wounds, burns and trophic ulcers. The inventive monolayer coating is embodied in the form of a film or gel and consists of rubber latex in a quantity of 25-98 mass% and at least one type of water-soluble ethyl alcohol-treated vegetable polysaccharide in a quantity of 2-75 mass%. The inventive method for producing said coating consists in mixing a rubber latex and a saccharide aqueous solution pretreated by ethyl alcohol in such a way that a homogeneous state thereof is attainable, distributing the thus obtained matter along a flat surface and in drying it until a gel or film is obtained. The coating is well adhesive to a wound and is easily removable therefrom along with wound epithelisation and can be used directly in the form of a coating, for transferring skin fibroplasts to a traumatic surface or for the cultivation thereof.

## Description

### Field of Technology

The invention relates to medicine and is intended for use in medical practice, for closing and treating wounds, burns, trophic ulcers.

Moreover, it can be used as a substrate for cell culture and as a means for delivering the cells to wounds.

### Prior art

For a long time, medicine has been faced with the problem of obtaining artificial materials in the form of films or sheets that are suitable for use as temporary wound coverings, which are quite cheap and easy to manufacture, and that have suitable physical and chemical properties, similar to the properties of the skin and adapted to approximate by their biological characteristics the features of such biological coatings, as human or pig skin, without such typical drawbacks, such as cost, biological incompatibility, complexity of sterilization and long-term preservation, impossibility to build up strategic reserves.

Currently, the problem is most successfully solved by coverings that are made of sheets or films of a mixture of synthetic polymers and of polymers of biological origin; In fact, coverings that are made only of synthetic polymer material, in particular one-component synthetic polymer material, such as polyurethane (US patent 2871218), or of multicomponent synthetic polymer material, such as a mixture of polyurethane and polyallyl ether (patent DE 34098558), have good physical and mechanical properties and are quite strong and resistant to water and steam, but only have a protective function and are not suitable for cell culture.

On the other hand, coverings made only of biopolymers, in particular one-component biopolymer material, such as collagen (US patent 4578067), or multicomponent biopolymer material, for example a mixture of gelatin and chitosan (US patent 4572906), or a mixture of collagen, chitosan and gycosaminoglycans (int. pat, appl. WO 88/10123), well stimulate the regeneration process and are suitable for cell culture, but have poor physical and mechanical properties: in fact they are brittle in the dry state, and poorly resistant in the moist state, and they can be quickly destroyed under the action of enzymes that are present in the biological fluids of the wound, in such a way that they cease both serving their protective function and working as regeneration stimulators.

The present invention relates to composite coverings for wounds containing polymers and polysaccharides. A wound covering is known (US patent 4524064), which has the form of a grid made of a water-insoluble hydrogel containing Polyvinyl alcohol in a quantity set between 1.5 and 8%, a polyatomic alcohol C2-C20 in a quantity set between 10 and 85% and a highly water-soluble substance in a quantity set between 0.2 and 15%, wherein the latter may be a polysaccharide, such as alginic acid, sodium alginate, carboxymethylcellulose, methylcellulose, carrageenan, dextrin, etc.

This coating is prepared: by mixing water solutions of the ingredients until a homogeneous state is achieved, by distributing the mixture on a flat surface having a number of protrusion per square meter set between 30 thousand and 200 thousand, by freezing the mixture and then hardening it with a subsequent vacuum-drying step, such that a dehydration is achieved, set between 5 and 95%. However, such hydrogels, when exposed to air, dry up quickly and get brittle. They are not suitable for long-term storage. A penetration of microbes can occur by pores towards the wound, but the pores close owing to the swelling of the polysaccharide component, and the vapor-liquid equilibrium of the wound is altered. Due to the strong water absorption in the first hours after the hydrogels, a crust develops on the wound. Moreover, this covering is not suitable for cell culture.

A covering for wounds is known (Swiss patent nr. 655662), which is in the form a of a film, reinforced with small fillers particles or grid material, based on a) a sewn hydrophilic polymer selected from the group comprised of: - an amide of acrylic or methacrylic acid; - an ester of acrylic or methacrylic acid and a polyatomic C2-C20 alcohol having free hydroxyl groups, in a percentage set between 50 and 90%; and b) a gelatin macromolecular material in a percentage set between 10 - 50%, for example, polysaccharide-agarose. This films are produced as follows: preparing a mixture of raw materials (such as acrylic acid amide) with alcohol, which is mixed with a water solution of a gelatin-forming macromolecular material; building up a layer of this mixture until a desired thickness of the film is reached; introducing the initiators of polymerization and carrying out the film-forming reaction; reinforcing the film with small fillers particles or grid material; and drying.

The need of reinforcing fillers or grid material is due to a lack of mechanical strength of such films. Besides, there is a risk of radication of the regenerating tissue in the film due to high adhesion of the hydrophilic synthetic polymer to the wound moist and to adhesion of the hydrophilic biopolymer to the proliferating cells.

A film is known, which is made of a mixture of collagen and natural rubber latex (certificate of authorship SU 1251528) and is intended for the cultivation of animal cells. This film has one-layer and is homogeneous in structure. However, it does not have the suitable adhesion properties towards fresh and bleeding wounds, and cannot be used as a wound covering. Furthermore, the presence of a protein component of a collagen increases the possibility of allergic reactions.

A film is known that is made of natural rubber latex (certificate of authorship SU 1691391) and is intended for animal cells culture. This film is homogenous in structure. However, it is absolutely impervious to water, it does not have the necessary adhesion properties, especially to fresh and bleeding wounds, and cannot be used as a wound covering.

A wound covering is known, which is in the form of a one-layer homogeneous film, made of a mixture of a fluorinated rubber latex and of vegetal origin polysaccharide (Russian patent 2091082). The method for obtaining such a film (Russian patent 2091082) provides the step of preparing a mixture of fluorinated polymer latex and of a polysaccharides water solution, which is arranged on a flat surface and then is dried. This film is elastic, adheres very well to the wound surface, but the fibroblasts that are deposed on it do not adhere to it, and hence they cannot reproduce since the physical conditions of the surface of this film does not allow fibroblasts to adhere to it.

A wound covering is known, which is in the form of a one-layer homogeneous film, that is made of a mixture of rubber latex, vegetal origin polysaccharides, collagen and chitosan, which allows treating wounds, burns, trophic ulcers, and can also be used as a substrate for cell culture (Russian patent 2193895). The method of obtaining such a film (Russian patent 2193895) provides the steps of preparing a mixture of rubber latex, of an aqueous polysaccharides solution, a chitosan solution and acidified solution of collagen, which are arranged in the form of monolayer on a flat surface and dried. However, the presence in the film animal-origin polysaccharides - chitosan, and of protein components - collagen, increases the possibility of allergic reactions.

### Disclosure of the invention

The main technical object, that the proposed invention aims to achieve, is to provide a one-layer covering for wounds in the form of a film or of a gel, which is easy to manufacture and is suitable for use as a substrate for delivering skin fibroblasts to a wound, or a substrate for cell culture. Another object is to reduce the possibility of allergic effects.

The stated objects are achieved by the fact that a one-layer covering for a wound is proposed, comprising rubber in the form of a latex, and a vegetal polysaccharide which, according to the invention, comprises, as a polysaccharide component, a polysaccharide previously treated with ethanol, in the following proportions related to the dry state of the coating as weight percentage: rubber 25-98%; polysaccharide 0.2-75%; and is obtained in the form of a film or of a gel with a dry matter content set between 1,5 and 98% in weight.

The coating comprises, as a rubber: a natural rubber, or a fluorine rubber, or a silicone (siloxane) rubber, or a butyl rubber or a styrene rubber, or a polyisobutylene, or another rubber, suitable for medical or food use, non-toxic to animal cells, that may be in the form of latex.

The covering can comprise , as a fluorine rubber, a copolymer of vinylidene fluoride with hexafluoropropylene or with trifluorchlorethylene.

As a polysaccharide component, the covering can comprise at least one polysaccharide, selected from the group comprised of: a water-soluble derivative of cellulose; pectin; agarose; alginic acid and its derivatives; carrageenan.

As a vegetal polysaccharide, the covering comprises preferably a water-soluble derivative of cellulose, for example: methylcellulose or carboxymethylcellulose, or a combination thereof.

Preferably, as a polysaccharide component the covering can comprise a mixture of at least two polysaccharides, selected from the group comprised of: methylcellulose; alginic acid and its derivatives; pectin, in any combination and ratio.

As a further polysaccharide component, the coating may contain a gel-forming vegetal resin, in particular, gum arabic, or xanthan gum, or tragacanth.

The objects are still solved by the fact that a method is proposed for obtaining a one-layer covering for wounds, comprising the steps of: preparation of a mixture of a rubber latex and of a polysaccharides water solution; formation of a layer of said mixture; drying said layer in the air, wherein, according to the invention, for said step of preparation of said mixture, a polysaccharide previously treated with ethanol is used, while said drying is carried out until the formation of a film or of a gel.

In a preferred embodiment, an ethanol treatment of said polysaccharide treated with ethanol is carried out with ethanol at a concentration set between 32% and 96% for 5 to 50 minutes, at room temperature.

Preferably, said treatment is carried out with ethanol at a concentration of 70% for 30 minutes.

Besides, as a rubber, a fluorine rubber is used, or a silicone rubber (siloxane), or a butyl rubber, or a natural rubber, or a styrene rubber, or a polyisobutylene, or another rubber, suitable for medical or food use, non-toxic to animal cells, that may be in the form of latex and may form a hydrophobic film.

Preferably, as a fluorine rubber, a copolymer of vinylidene fluoride with hexafluoropropylene or trifluorchlorethylene is used.

As a vegetal polysaccharide, at least one polysaccharide is used, said polysaccharide selected from the group comprised of: a water-soluble derivative of cellulose; pectin; agarose; alginic acid and its derivatives; carrageenan.

Preferably, as a vegetal polysaccharide, water-soluble derivatives of cellulose are used, in particular, methylcellulose or carboxymethylcellulose, or a combination thereof.

As a polysaccharide component, a mixture of at least two polysaccharides is also used, said polysaccharides selected from the group comprised of: methylcellulose; alginic acid and its derivatives; pectin, in any combination and ratio.

As an additional polysaccharide component, a gel-forming vegetal resin is used, for example, gum arabic, or xanthan gum or tragacanth.

It has been found experimentally that the one-layer covering for wounds obtained in the form of a film or of a gel, when prepared from rubber latex and ethanol-treated vegetal polysaccharides, becomes suitable for adhesion to the skin fibroblasts and thus can be used as a substrate for the cultivation of animal cells, in particular skin fibroblasts. An explanation of this phenomenon may be that, in the treatment of polysaccharides with ethanol at the above-mentioned concentrations, the degree of dispersion of the aqueous solution of polysaccharides subsequently obtained is significantly increased, which improves the physical properties of coating surface, making it suitable for skin fibroblast cell adhesion. This way, the covering retains a good steam permeability and adhesion to the wound, it remains transparent. A covering that is used as a film retains flexibility and does not break when bending. This cover can be used for treating wounds, burns, trophic ulcers, as well as for carrying cells in skin transplantation.

The use of alcohol at a concentration less than 32% undesirably increases the treatment time of polysaccharides, while, at a concentration higher than 96%, an unwanted dehydration of polysaccharides may occur.

Hereinafter, some exemplary embodiments of the covering according to the invention are presented, to allow a better understanding of the invention.

### Alternative ways to carry out the invention.

To obtain the proposed wound coverings are used:
- Natural rubber latex, for example, type Reverteh T -"Spravo 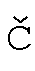 nik rezin 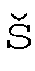 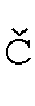 ika", Medicinskaja Himija, 1971, p.21, 200;
- Polyisobuthylene latex - "Spravo 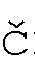 nik rezin 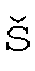 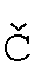 ika", Medicinskaja Himija, 1971, pp. 186, 190
- Siloxane Rubber CKT latex - "Spravo 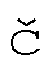 nik rezin 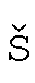 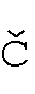 ika", Medicinskaja Himija, 1971, p.137;
- Latex of a copolymer of hexafluoropropylene and with vinylidene fluoride (trademark in Russia "SKF-26" similar to "Viton", USA, "Spravo 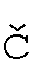 nik rezin 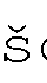 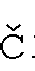 ika", Medicinskaja Himija, 1971, pp. 150 246), production of Kirovo- 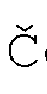 epeckogo Chemical Plants (TU 6-05-04-352-83 amended 1) hereinafter CKF-26;
- Latex of a copolymer of trifluoroethylene with vinylidene fluoride (trademark in Russia "CKF-32", in the USA "Kel-F ","Spravo 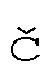 nik rezin 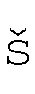 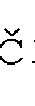 ika ", Medicinskaja Himija, 1971, pp. 150 46) production Kirovo- 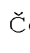 epeckogo Chemical Plants (TU 6-05-65-137), hereinafter CKF-32, which are rubber medical supplies;
- Water-soluble Methylcellulose, M0262 - Directory "Reagents for Biochemistry and research in the sciences" SIGMA, 1999, p.694;
- Water-soluble Methylcellulose brand MTS 100, produced of "Himprom, Usol'e-Sibirskoe (TU 6-01-717-72 Change. 1,2,3); - Carboxymethylcellulose, C5672 - Directory "Reagents for Biochemistry and research in the sciences" SIGMA, 1999, p.22;
- Alginic acid, A7003 - Directory "Reagents for Biochemistry and research in the sciences" SIGMA, 2004-2005, p.116;
- Sodium alginate, medical grade (VFS 42-1680-87) or sodium alginate, food grade (TU 6-09-10-535), derived from brown algae in the Arhangel'sk algal plant;
- Sodium alginate, A2033 - Directory "Reagents for Biochemistry and research in the sciences" SIGMA, 2004-2005, p.116;
- Carrageenan, S1263 - Directory "Reagents for Biochemistry and research in the sciences" SIGMA, 2004-2005, p.388;
- Pectin, P9135 (from citrus) - Handbook "Reagents for Biochemistry and research in the sciences" SIGMA, 1999, p.790;
- Gum arabic, G9752 - Directory "Reagents for Biochemistry and research in the sciences" SIGMA, 1999, p.526;
- Xanthan gum, G 1253 - Directory "Reagents for Biochemistry and research in science " SIGMA, 1999, p.526;
- Tragakant, G1128 - Directory "Reagents for Biochemistry and research in the sciences" SIGMA 5 1999, p.526.

The coating according to the invention is obtained as follows.

Vegetal origin dry polysaccharides are sprayed with a 32 to 96% ethylic alcohol solution, then they are matured for 5 to 50 min. at room temperature, then they are decanted and the liquid remaining over the solid is drained.

From the so treated polysaccharides, an aqueous solution is prepared, in which a predetermined amount of rubber latex (with a concentration of dry matter ranging from 15 to 60%) is added, then it is preliminary filtered from coagulated particles by percolation through Nylon filters having mesh of 1.0, 0.5, 0.2 and 0.1 mm, and then thoroughly mixed to obtain a homogenous mass. The mixture is poured in a cup or in a glass plate to form a layer, whose thickness is calculated depending on the concentration of dry matter in the covering, and dried at room temperature to obtain a gel with the specified dry matter or dry film. The covering is cut into sheets of desired size and shape, then it is tightly packed and sterilized by a gamma radiation dose of 2.5 Mrad.

We reproduce herewith examples of embodiments and applications of the wound covering:

### Example 1.

To obtain the covering according to the invention, 20 g methylcellulose were treated with 70% ethanol for 30 minutes, then they were decanted, and the liquid remaining over the solid was drained. From the ethanol-treated methylcellulose, 1 liter of 2% water solution was produced, to which 0.4 liters of pre-strained latex fluorine rubber (Mark CKF-26) were added, containing 360 grams per liter of polymer in terms of dry matter.

All the components are thoroughly mixed to obtain a homogeneous mass, which was percolated through a nylon filter with a mesh of 0.2 and 0.1 mm. The mixture was cast on a glass plate and dried by exposure to air at room temperature, to obtain an elastic film. Then the film was tightly packaged and sterilized by a gamma radiation dose of 2.5 Mrad.

### Example 2.

10 g carboxymethylcellulose were treated with 50% ethanol for 45 minutes, then they were decanted, the liquid remaining over the solid was drained and a 3% water solution was produced, which was mixed with 0.4 liters of pre-strained rubber latex siloxane containing 300 grams per liter of polymer. Afterwards, a film was produced as described in Example 1.

### Example 3.

10 g pectin and 10 g methylcellulose were treated with 90% ethanol during 10 minutes, then they were decanted, the liquid remaining over the solid was drained and a 2% water solution of polysaccharides was produced, which was mixed with 0.5 liters of pre-strained copolymer latex hexafluoropropylene and vinylidene fluoride containing 300 grams per liter of polymer. Afterwards, a film was produced as described in Example 1.

### Example 4.

5 g sodium alginate, 5 g carrageenan and 10 g methylcellulose were treated with 50% ethanol for 40 minutes, then they were decanted, the liquid remaining over the solid was drained and a 2% water solution of polysaccharides was produced, which was mixed with 0.5 liters of previously filtered natural rubber latex containing 200 grams per liter of dry matter. Afterwards, a film was produced as described in Example 1.

### Example 5.

0.2 g of gum arabic, 0.3 g xanthan gum, 5 g pectin and 10 g carboxymethylcellulose were treated with 90% ethanol during 7 minutes, then they were decanted, the liquid remaining over the solid was drained and a 2% water solution was produced and mixed with a polysaccharide, which was mixed with 0.5 liters of pre-strained rubber latex acrylic containing 200 grams per liter of polymer. Afterwards, a film was produced as described in Example 1.

### Example 6.

20 g carboxymethylcellulose, 0.2 g of gum arabic, 0.2 g xanthan gum, were treated with 70% ethanol during 30 minutes, then they were decanted, the liquid remaining over the solid was drained and a 2% water solution of polysaccharides was produced, which was mixed with 0.5 liters of pre-strained latex polyisobuthylene, containing 300 grams per liter of polymer. Afterwards, a film was produced as described in Example 1.

### Example 7.

20 g methylcellulose, 10 g pectin and 5 grams agarose were treated with 50% ethanol for 40 minutes, then they were decanted, the liquid remaining over the solid was drained and a 2% water solution of polysaccharides was produced, which was mixed with 0.5 liters of pre-strained natural rubber latex containing 300 g/l polymer. Afterwards, a film was produced as described in Example 1.

### Example 8.

5 g carrageenan, 10 g sodium alginate and 10 g methylcellulose were treated with 70% ethanol during 30 minutes, then they were decanted, the liquid remaining over the solid was drained and a 2% water solution of polysaccharides was produced, which was mixed with 0.5 liters of pre-strained latex fluorine rubber (Russian trademark CKF-32) containing 300 g/l dry matter. Afterwards, a film was produced as described in Example 1.

### Example 9.

10 g methylcellulose, 10 g sodium alginate, 2 g pectin, 2g carrageenan and 0.2 g xanthan gum were treated with 96% ethanol for 15 minutes, then they were decanted, the liquid remaining over the solid was drained and a 2% aqueous solution of polysaccharides was produced, which was mixed with 0.5 liters of pre-strained latex of a copolymer of hexafluoropropylene with vinylidene fluoride containing 300 grams per liter of polymer. Afterwards, a film was produced as described in Example 1.

### Example 10.

To obtain the covering according to the invention, 20 g methylcellulose were used and treated with 70% ethanol for 30 minutes, then they were decanted and the liquid remaining over the solid was drained. From the ethanol-treated methylcellulose were produced 0.4 liters of 2% water solution, to which 1 l of pre-strained fluorine rubber latex (Russian trademark CKF-26) was added, containing 360 grams per liter of polymer as dry matter.

All the components are thoroughly mixed to obtain a homogeneous mass, which was percolated through a nylon filter with a mesh of 0.2 and 0.1 mm. The mixture was cast on a glass plate and dried by exposure to air at room temperature, to obtain a gel with containing 70% weight of dry matter. Then the gel was tightly packed and sterilized by a gamma radiation dose of 2.5 Mrad.

### Example 11.

10 g carboxymethylcellulose were treated with 50% ethanol for 45 minutes, then they were decanted, the liquid remaining over the solid was drained and a 3% water solution was produced, that was mixed with 0.4 liters of pre-strained rubber latex siloxane containing 300 grams per liter of polymer. Afterwards, a gel was prepared, as described in Example 10, with 75% weight of dry matter.

### Example 12.

10 g pectin and 10 g methylcellulose were treated with 90% ethanol during 10 minutes, then they were decanted, the liquid remaining over the solid was drained and a 2% water solution of polysaccharides was produced, which was mixed with 0.4 liters of pre-strained copolymer latex hexafluoropropylene and vinylidene fluoride containing 300 grams per liter of polymer. Afterwards, a gel was prepared, as described in Example 10, with 65% weight of dry matter

### Example 13.

5 g sodium alginate, 5 g carrageenan and 10 g methylcellulose were treated with 50% ethanol for 40 minutes, then they were decanted, the liquid remaining over the solid was drained and a 2% water solution of polysaccharides was produced, which was mixed with 0.5 liters of pre-strained natural rubber latex containing 200 grams / l dry matter. Afterwards, a gel was prepared, as described in Example 10, with 65% weight of dry matter.

### Example 14.

0.2 g of gum arabic, 0.3 g xanthan gum, 5 g pectin and 10 g carboxymethylcellulose were treated with 90% ethanol during 7 minutes, then they were decanted, the liquid remaining over the solid was drained and produced 2% water solution mixed with a polysaccharide which is 0.5 liters of pre-strained rubber latex acrylic containing 200 grams per liter of polymer. Afterwards, the gel was prepared, as described in Example 10, with 70% weight of dry matter.

### Example 15.

20 g carboxymethylcellulose, 0.2 g of gum arabic, 0.2 g xanthan gum were treated with 70% ethanol during 30 minutes, then they were decanted, the liquid remaining over the solid was drained and a 2% water solution of polysaccharides was produced, which was mixed with 0.5 liters of pre-strained latex polyisobuthylene containing 300 g/l polymer. Afterwards, a gel was prepared, as described in Example 10, with 85% weight of dry matter.

### Example 16.

20 g methylcellulose, 10 g pectin and 5 g agarose were treated with 50% ethanol for 40 minutes, then they were decanted, the liquid remaining over the solid was drained and a 2% water solution of polysaccharides was produced, which was mixed with 0.5 liters of pre-strained natural rubber latex containing 300 g/l. polymer. Afterwards, a gel was prepared, as described in Example 10, with 65% weight of dry matter.

### Example 17.

5 g carrageenan, 10 g sodium alginate and 10 g methylcellulose were treated with 70% ethanol during 30 minutes, then they were decanted, the liquid remaining over the solid was drained and a 2% water solution of polysaccharides was produced, which was mixed with 1 l pre-strained latex fluorine rubber (Mark CKF-32) containing 300 g/l dry matter. Afterwards, a gel was prepared, as described in Example 10, with 75% weight of dry matter.

### Example 18.

10 g methylcellulose, 10 g sodium alginate, 2 g pectin, 2g carrageenan and 0.2 g xanthan gum were treated with 96% ethanol for 15 minutes, then they were decanted, the liquid remaining over the solid was drained and produced 2% aqueous solution of polysaccharides, which was mixed with 0.5 liters of pre-strained Latex copolymer hexafluoropropylene and vinylidene fluoride containing 300 grams per liter of polymer. Afterwards, a gel was prepared, as described in Example 10, with 50% weight of dry matter.

The physical, mechanical and biological properties of the covering, according to the invention, were studied, in comparison with a prototype (Russian patent nr. 2193895), with similar products (Russian patent nr. 2091082) and with culture dish, such as the material traditionally used for the cultivation of animal cells, including human skin fibroblasts. As a culture dish, a ø 3.5 cm size Petri dish was used of the type Nunclon Surface by Nunc, Denmark. Steam permeability was determined according to GOST 838.17. Adhesion properties were investigated in terms of adhesion percentage on gauze, according to GOST R 15013.

The biological properties of the coating were studied by using it as a substrate in the cultivation of human skin fibroblast cells in a "needle" medium, in the manner described in the monograph by B.K. Gavriljuk, et al. "The culture of cells and tissue reconstruction (in the case of skin)", USSR Academy of Sciences, NCBI, IBF, Pu 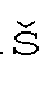 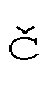 ino, 1988, p.104. The results of the study are presented in Tables 1 and 2. As it can be seen in Table 1, the covering obtained in the form of a film, has a steam permeability ranging from 2,5 to 5,0 mg/cm²/hr. The covering realized in the form of a gel, has a steam permeability ranging from 4,0 to 5,0 mg/cm²/hr (see table 2).

Adhesion to the wounds surface of 35-45% of adhesion on Gauze (see table 1, 2).

By using the covering according to the invention as a substrate for transporting human skin fibroblast cells in a "needle", 80 to 95% of the seeded cells had been flourishing one day after seeding, while the cells produced at the second day of culture were 100% to 120% of the seeded cells, for the coverings used in the form of a film (see Table 1).

About the covering used in the form of a gel, 90% to 100% of the seeded cells had been flourishing one day after seeding, and the cells produced at the second day of culture were 105-120% of the seeded cells (see Table 2).

Thus, the covering according to the invention for wounds has good steam permeability and adhesion properties, and can also be used as a substrate for the cultivation of skin cells, such as fibroblasts.

**TABLE 1**

| **EXAMPLES** | **PHYSICAL, MECHANICAL AND BIOLOGICAL PROPERTIES** | | | | |
|---|---|---|---|---|---|
| Covering in the form of a film | Steam permeability mg/cm²/hr | Adhes. to wound surface (% of total Gauze adhesion) | Phibroblast number (thousand/cm2) | Cells fluorishing after 24 hr (thousand / cm²) | Cells yield at 2nd day (thousand / cm²) |
| Example 1 | 3,5 | 45 | 100 | 90 | 110 |
| Example 2 | 2,5 | 40 | 100 | 85 | 115 |
| Example 3 | 4,5 | 40 | 100 | 90 | 110 |
| Example 4 | 5,0 | 35 | 100 | 80 | 105 |
| Example 5 | 4,5 | 45 | 100 | 95 | 120 |
| Example 6 | 3,5 | 45 | 100 | 95 | 105 |
| Example 7 | 5,0 | 40 | 100 | 92 | 105 |
| Example 8 | 5,0 | 45 | 100 | 95 | 115 |
| Example 9 | 4,5 | 40 | 100 | 95 | 105 |
| Prototype RU2193895 | 2,0 - 5,0 | 25 - 45 | 200 | 50-100 | 150 - 200 at 10^{th} day |
| Similar products RU2091082 | 2,0 - 5,0 | 25 - 45 | 200 | 40 - 50 | 20-25 at 10^{th} day |
| Culture dish | - | | 100 | 95 | 120 |

**TABLE 2**

| **EXAMPLES** | **PHYSICAL, MECHANICAL AND BIOLOGICAL PROPERTIES** | | | | |
|---|---|---|---|---|---|
| Covering in the form of a gel | Steam permeability mg/cm²/hr | Adhes. to wound surface (% of total Gauze adhesion) | Phibroblast number (thousand/cm2) | Cells fluorishing after 24 hr(thousand / cm²) | Cells yield at 2nd day (thousand / cm²) |
| Example 10 | 4,5 | 50 | 100 | 95 | 120 |
| Example 11 | 4,0 | 45 | 100 | 98 | 115 |
| Example 12 | 5,0 | 45 | 100 | 100 | 115 |
| Example 13 | 5,0 | 40 | 100 | 98 | 110 |
| Example 14 | 4,5 | 45 | 100 | 95 | 110 |
| Example 15 | 4,0 | 45 | 100 | 95 | 120 |
| Example 16 | 5,0 | 45 | 100 | 100 | 110 |
| Example 17 | 5,0 | 45 | 100 | 100 | 115 |
| Example 18 | 5,0 | 45 | 100 | 95 | 105 |
| Prototype RU 2193895 | 2,0 - 5,0 | 25 - 45 | 200 | 50 - 100 | 150 - 200 at 10^{th} day |
| Similar products RU 2091082 | 2,0 - 5,0 | 25 - 45 | 200 | 40-50 | 20-25 at 10^{th} day |
| Culture dish | - | - | 100 | 95 | 120 |

### Industrial applicability

The exemplary specific embodiments confirm that the proposed one-layer covering for wounds in the form of a film or of a gel can be obtained by the proposed method.

In comparison with a similar product (Russian patent nr. 2091082) the coating according to the invention can be used for treatment the wounds, as well as a film-substrate for a cell culture.

In comparison with a prototype (Russian patent 2193895), the covering according to the invention does not contain components of animal origin, and therefore does not cause allergic reactions due to these components.

In addition, the polysaccharide part of the coating, which is hydrophilic, interacts with poly-cations of the wound surface and/or of the exudate of the wounds and partly dissolves in it. On the one hand, this leads to an increase in affinity of the fibroblasts, which are on the covering, as well as on the substrate, for the wound surface and for their mixing on the wound surface. On the other hand, it also leads to a more easy detachment of the remaining, hydrophobic part of the covering, the molecules of the rubber, which increases the possibility of withdrawing the covering from the wound surface without trauma.

## Claims

1. One-layer covering for wounds comprising a rubber latex and a water-soluble polysaccharide of vegetal origin **characterised in that** said one-layer covering comprises, as a polysaccharide component, a polysaccharide previously treated with ethanol, with the following ratio of components in terms of dry matter, by weight percentage:
- rubber 25 - 98%,
- polysaccharides 2 - 75%, said one-layer covering being made in form of a film or of a gel with a content of dry matter set between 2.0 and 98.0 per cent by weight.

2. One-layer covering according to claim 1, comprising, as a rubber, a fluorine rubber or a silicone rubber or a natural rubber, or a butyl rubber or a styrene rubber, or a polyisobutylene, or another rubber, suitable for medical or food use, non-toxic to animal cells, in particular, in the form of latex.

3. One-layer covering comprising, as a fluorine rubber, a copolymer of vinylidene fluoride with hexafluoropropylene or trifluorchlorethylene.

4. One-layer covering according to claim 1, comprising, as a polysaccharide component, at least one polysaccharide, selected from the group comprised of: a water-soluble derivative of cellulose; pectin; agarose; alginic acid and its derivatives; carrageenan.

5. One-layer covering according to claim 1, comprising, as a polysaccharide component, a water-soluble derivatives of cellulose, in particular, methylcellulose or carboxymethylcellulose, or a combination thereof.

6. One-layer covering according to claim 1, comprising, as a polysaccharide component, a mixture of at least two polysaccharides, selected from the group comprised of methylcellulose; alginic acid and its derivatives; pectin, in any combination and ratio.

7. One-layer covering according to claim 4, comprising a further polysaccharide component - a gel-forming vegetal resin, in particular, gum arabic, or xanthan gum or tragacanth.

8. A method for obtaining a one-layer covering for wounds, comprising the steps of: preparation of a mixture of a rubber latex and of a water solution of vegetal polysaccharides; formation of a layer of said mixture; drying said layer in the air, **characterised in that** in said step of preparation of said mixture a polysaccharide previously treated with ethanol is used, while said drying is carried out until the formation of a film or of a gel.

9. A method according to claim 8, wherein an ethanol treatment of said polysaccharide treated with ethanol is carried out with ethanol at a concentration set between 32 and 96% for 5-50 minutes.

10. A method according to claim 8, wherein, as a rubber, a fluorine rubber is used, or a silicone rubber, or a natural rubber, or a butyl rubber, or a styrene rubber, or a polyisobutylene, or another rubber, suitable for medical or food use, non-toxic to animal cells, in particular in the form of latex.

11. A method according to claim 10, wherein, as a fluorine rubber, a copolymer of vinylidene fluoride with hexafluoropropylene or trifluorchlorethylene is used.

12. A method according to claim 8, wherein, as a vegetal polysaccharide, at least one polysaccharide is used, said polysaccharide selected from the group comprised of water-soluble derivatives of cellulose; pectin; agarose; alginic acid and its derivatives; pectin.

13. A method according to claim 8, wherein, as a vegetal polysaccharide, water-soluble derivatives of cellulose of are used, in particular, methylcellulose or carboxymethylcellulose, or a combination thereof.

14. A method according to claim 8, wherein, as a vegetal polysaccharide, a mixture of at least two polysaccharides is used, said polysaccharides being selected from the group comprised of: methylcellulose; alginic acid and its derivatives; pectin, in any combination and ratio.

15. A method according to claim 12, wherein, as a vegetal polysaccharide, a gel-forming vegetal resin is additionally used, in particular, gum arabic, or xanthan gum or tragacanth.
